# EUROPEAN PATENT SPECIFICATION

(11) **EP 3 703 588 B1**
(45) Date of publication and mention of the grant of the patent: **17.04.2024**
(21) Application number: 18874260.5
(22) Date of filing: 25.10.2018
(51) Int. Cl.: A61B 17/128, A61B 17/29, A61B 17/00

(54) **SURGICAL APPLIANCE**
CHIRURGISCHES GERÄT
APPAREIL CHIRURGICAL

(30) Priority: 05.11.2017 US 201762581729 P
(43) Date of publication of application: 09.09.2020
(73) Proprietor: Grena USA LLC, Wilmington, DE 19801 (US)
(72) Inventor: BRODACZEWSKI, Wieslaw Mieczyslaw, Brentford TW8 8ER (GB); DECEWICZ, Andrzej Janusz, Nottingham NG3 4HQ (GB); WAWRYNIUK, Grzegorz Andrzej, 02-495 Warsaw (PL)
(74) Representative: Pure Ideas Limited
(86) International application number: PCT/US2018/057439
(87) International publication number: WO 2019/089331

(56) References cited:
- EP-A1- 1 810 625
- EP-A2- 2 823 775
- WO-A1-03/030741
- WO-A1-2006/071120
- WO-A1-2016/200496
- US-A- 5 290 308
- US-A1- 2009 088 792
- US-A1- 2012 016 391
- US-B1- 7 431 188

## Description

### Technical field

The present invention relates generally to surgical instruments, and more specifically, to devices for applying ligation clips.

### Background technique

During laparoscopic and endoscopic surgery, the surgeon must often terminate the flow of blood or other fluids through one or more blood vessels or catheters. Ligation clips have been applied to blood vessels or catheters to prevent body fluids from flowing through the blood vessels or catheters. Endoscopic clips using surgical instruments have been used to apply ligature clips, such as polymer ligature clips. Such ligature clips are usually made of biocompatible materials and are usually compressed on blood vessels or catheters. Once applied to the blood vessel or catheter, the compressed clip will terminate the flow of fluid through the blood vessel or catheter.

The clamp for applying surgical instruments usually includes an actuator jaw located at the distal end of the barrel and a handle portion located at the proximal end of the barrel. The safety mechanism for actuating the actuator jaws is located near the proximal end.

Endoscopic or laparoscopic surgery is usually performed away from the incision. Therefore, the application of the ligature clip must be completed by the surgeon's reduced field of view or reduced tactile feedback. Therefore, it is desirable to improve the operation of surgical instruments by providing an instrument that has enhanced maneuverability and can be articulated in multiple directions without requiring the practitioner to use two hands and avoiding inadvertent actuation of the actuator jaws.

European Patent Application EP 1 810 625 A1 discloses an endoscopic bipolar forceps including a housing having a shaft affixed thereto, the shaft including jaw members at a distal end thereof. The shaft is rotatable relative to a handle. The forceps include a rotating assembly to rotate the shaft. The rotating assembly is equipped with one or more mechanical interfaces that are rotatable with or within the rotating assembly and that are configured to produce tactile and/or audible feedback to the user during rotation. This document does not disclose that the rotating assembly comprises ring gear received in a sleeve and a detent latch extending radially through the sleeve, wherein the detent latch engages the ring gear.

International Patent Application WO 03/030741 A1 discloses an incremental rotational displacement mechanism. The mechanism includes a circular detent housing having a plurality of detents arranged along the circumference of the detent housing. A detent ring is positioned coplanar to and within the circumference of the detent housing. At least a portion of at least one detent arm engages at least a portion of the detent housing. A handle may engage the detent ring, so that a force applied to the handle causes the detent ring to rotate with respect to the circular detent housing.

### Summary of the invention

Improved surgical instruments used for surgery such as applying polymer ligation clips are configured for enhanced maneuverability and one-handed operation. The surgical instrument includes a barrel with an actuator jaw mounted to a carrier, and the carrier is fixed to the distal end of the barrel. The handle part is positioned at the proximal end of the barrel. The handle part includes a grip for opening and closing the actuator jaws. The handle part also includes a knob for hingedly connecting the carrier, a knob for rotating the barrel, and a safety mechanism for automatically preventing accidental activation of the gripper.

From the foregoing outline, it will be appreciated that one aspect of the present invention is to provide surgical instruments with improved general characteristics described that are not affected by the previous foregoing disadvantages of the present invention.

The feature of the present invention is to provide a surgical instrument with improved general features that facilitate the application of the ligation clip.

The consideration of the present invention is to provide a surgical instrument with improved general characteristics described that enables the surgeon to ligate the blood vessel wall without excessive manipulation.

Aspects of the present invention are outlined in claim 1 below and other features in the claims dependent thereon.

Other aspects, features and considerations of the present invention will be obvious and will be partially pointed out below.

In view of these objectives, the present invention finds that the above-mentioned aspects, features and considerations and certain other aspects, features and considerations are achieved, or the scope of the present invention which will be more specifically pointed out and indicated in the appended claims with reference to the drawings and Examples of various combinations of elements, arrangements of parts, and a series of steps.

### Description of the drawings

In the drawings, one of various possible exemplary embodiments of the present invention is shown:

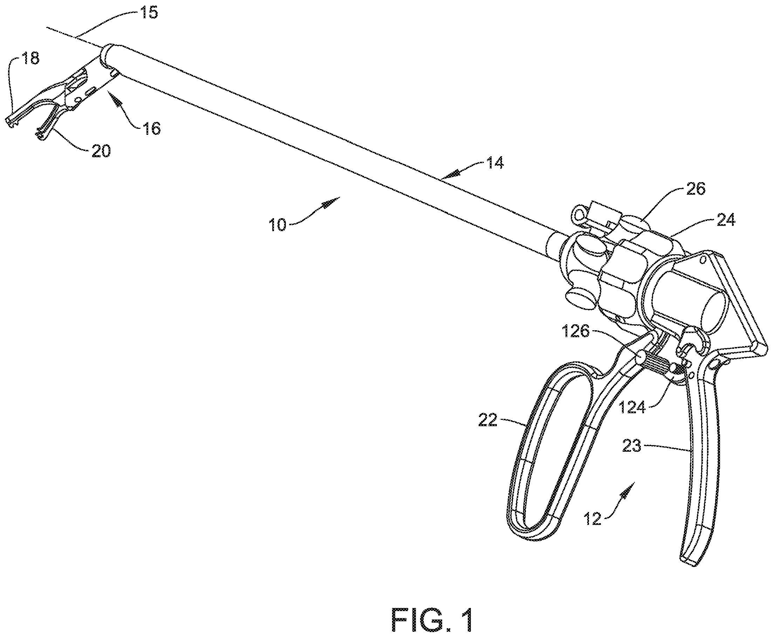

Figure 1 is an isometric view of a surgical instrument constructed in accordance with the present invention and illustrating the present invention, and shows a pair of actuator jaws mounted to a carrier located at the distal end of the barrel and located The handle part at the proximal end of the barrel;

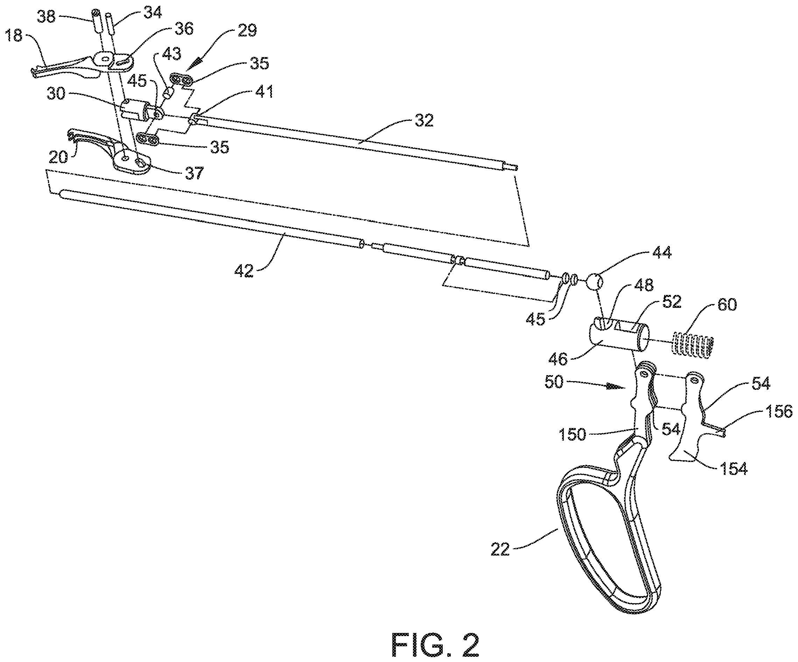

Figure 2 is an exploded view of components used to close and open the actuator jaws;
Figure 3 is a partial cross-sectional view showing the actuator jaw in an open position through a part of the surgical instrument;
Figure 4 is its side view;
Figure 5 is a partial cross-sectional view showing the actuator jaw in a closed position through a part of the surgical instrument;
Figure 6 is a side view of the jaws in the closed position;
Figure 7 is an exploded view of the part of the handle for closing and opening the actuator jaws in the direction in which the safety mechanism automatically eliminates accidental actuation of the actuator jaws;

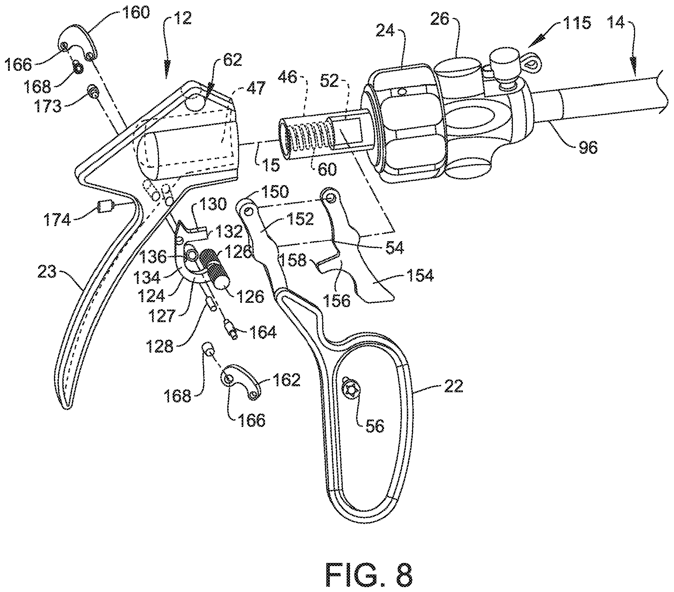

Figure 8 is an exploded view of the part of the handle for closing and opening the actuator jaws in which the safety mechanism is in the unlocking direction allowing repeated actuation of the actuator jaws;
Figure 9 is an exploded view of the hinged parts for the carrier;
Figure 10 is a partial cross-sectional view similar to Figure 4 through a part of the surgical instrument and showing a carrier coaxial with the barrel;
Figure 11 is a partial cross-sectional view through a part of the surgical instrument and showing the carrier in the hinged position;
Figure 12 is an exploded view of components used for the axial rotation of the cylinder;
Figure 13 is a partial side view of the surgical instrument with the safety mechanism in the automatic locking position;
Figure 14 is an enlarged view of the part circled in Figure 13;
Figure 15 is a partial side view of the surgical instrument in which the safety mechanism is unlocked; and
Fig. 16 is an enlarged view of the part circled in Fig. 15.

### Detailed description

The present invention will now be described in detail with reference to the accompanying drawings, which are provided as illustrative examples of the present invention to enable those skilled in the art to practice the present invention. It should be noted that the following drawings and examples are not meant to limit the scope of the present invention to a single embodiment, but other embodiments may be implemented by interchange of some or all of the described or illustrated elements.

Unless clearly stated, the applicant does not intend to give any term in the specification or claims an uncommon or special meaning.

Referring now to the drawings, the reference numeral 10 denotes a surgical instrument constructed in accordance with the present invention and illustrating the present invention, and the surgical instrument can be used for operations such as the application of polymer ligature clips. The appliance 10 includes a proximal handle portion 12, a hollow cylindrical main body 14 extending along the longitudinal axis 15, and a carrier 16 that carries a pair of actuator jaws 18, 20. The handle portion 12 includes a grip 22, a seat 23, a hinge button 24, and a barrel knob 26. The grip, seat, hinge button and barrel knob are positioned in close proximity to each other for one-handed manipulation For example, the gripping member 22 is squeezed and released by the fingers of the practitioner, wherein the palm of the practitioner is placed on the seat member 23, and the buttons 24 and 26 are rotated by the thumb and/or index finger of the practitioner.

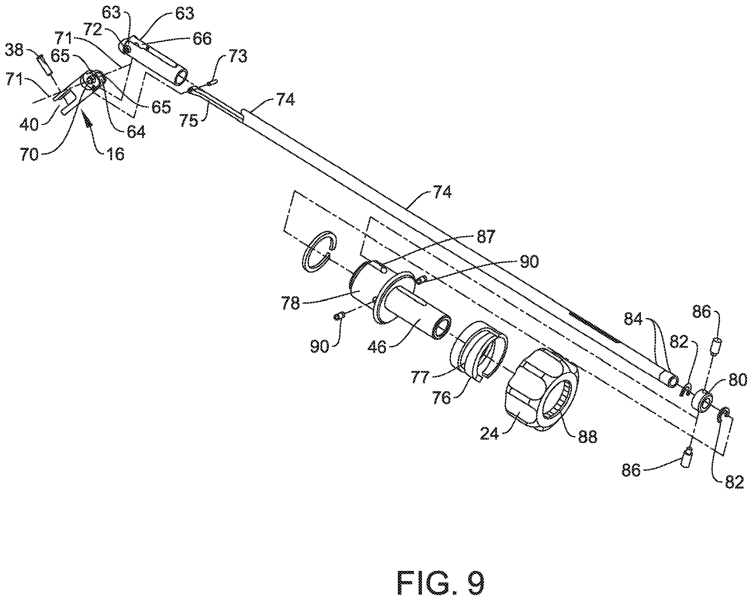

It should be noted that the jaws 18, 20 are actuated by the end U-shaped clip 30, the end U-shaped clip 30 is joined to the end of the rod 32 through a link 29, a single link of the link 29 and the roller chain The pieces are similar. The pivot 41 protrudes from the distal end of the rod 32 through the proximal holes of the pair of side plates 35, and the pin 43 extends through the distal holes of the side plates and the holes of the clevis flange 45. Additional pins 34 extend through opposite faces of the clevis 30 and engage with curved cam grooves 36, 37 in the jaws 18, 20, respectively. As shown in FIG. 9, when the U-shaped clamp 30 and the rod 32 perform linear movement, the jaws 18 and 20 pivot about the pin 38, and the pin 38 extends through the yoke 40 of the carrier 16. Part of the jaws 18 and 20 are received in the yoke 40.

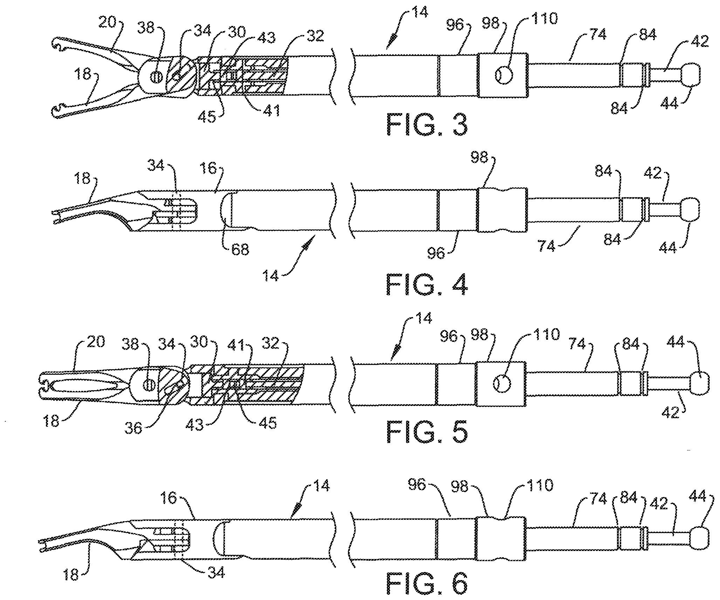

The proximal end of the rod 32 is fixed to the distal end of the tie rod 42, and the tie rod 42 includes a ball 44 at its proximal end. The cylindrical linear actuator 46 is housed in the hollow housing 47 of the handle portion 12, and the cylindrical linear actuator 46 includes a socket 48 that receives the ball 44. The main leg 50 of the grip 22 includes a welded laminate of three plates 150, 152 and 154 and a cam surface 54 received in the groove 52 of the linear actuator 46. It should be noted that when the gripping member 22 is squeezed toward the seat member 23, the main leg 50 pivots about the pin 56 fixed in the hole 62 of the handle portion 12, thereby causing the linear actuator 46 and The ball 44 moves in the proximal direction along the axis 15 so as to compress the return spring 60. Simultaneously, the pull rod 42, the rod 32, and the end U-shaped clip 30 move toward the proximal end, thereby causing the actuator jaws 18, 20 to move from the normally open position shown in FIG. 3 to the closed position shown in FIG. 5 mobile.

Referring now to FIG. 9, there is shown in FIG. 9 the components used to articulate the carrier 16, which includes a reduced thickness proximal channel flange 64 having a spaced apart The parallel plates 65 are received between parallel spaced apart walls 63 of the channel 66 formed in the head sleeve.

The pivot 70 protrudes from each plate 65 and is located in the hole 72 of the channel wall 63. The head sleeve is located in the distal end of the barrel 14.

The hinge connection of the carrier 16 about the rotation axis 71 concentric with the pivot shaft 70 is effected by the reciprocating axial movement of the hollow push rod 74 extending through the cylinder 14. The end portion of the leg 75 extending from the distal end of the push rod 74 is received between the plates 65. The pin 73 extends through the offset hole 79 of the plate 65 and the end portion of the leg 75. Therefore, the axial movement of the push rod 74 causes the articulation of the carrier 16 about the pivot 70 to take effect. The pull rod 42 extends through the hollow push rod 74, and a pair of gasket seals 45 are provided between the pull rod 42 and the push rod 74.

As shown in FIG. 9, by rotating the cylindrical cam 76 having the spiral passage 77, the rotation of the hinge knob 24 is converted into the axial movement of the push rod 74. The cam 76 is located on a collar 78 fitted on the distal end of the linear actuator 46. The collar 78 is assembled on a ring 80 that is fixed to the push rod 74 and is prevented from relative axial movement by a pair of clamps 82 located in corresponding grooves 84. A pair of radially opposed cam followers 86 are fixed to the ring 80 and engaged with the spiral passage 77 of the cam 76.

The follower 86 also extends into a pair of diametrically opposed passages 87 of the collar 78. Therefore, through the engagement between the cam follower 86 and the cylindrical cam 76, the rotation of the hinge knob 24 causes the axial movement of the push rod 74 and the pivotal movement of the carrier 16 about the axis of the pivot 70.

Located in the hinged button 24 is an internal gear 88 that rotates with the button 24. When the button 24 rotates, the teeth of the gear 88 are engaged by a pair of ball stopper latches 90 located in the diametrically opposed sockets of the collar 78, thereby increasing by a yieldable and audible limit stopper Spin.

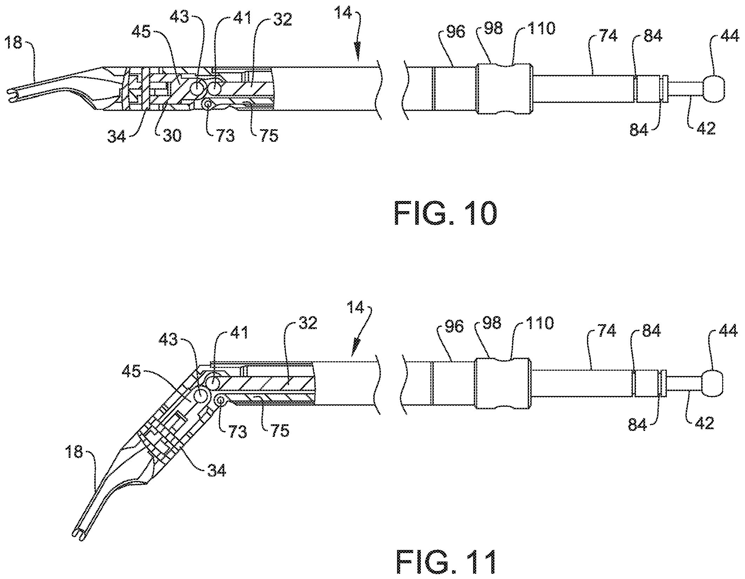

In order to increase maneuverability in the body cavity, it is desirable to minimize the size of the actuator jaws 18, 20 and the size of the carrier 16. By way of example, as shown in Figure 10, the length of each actuator jaw from its distal tip to the center of the pivot pin 38 will be on the order of 29mm and the carrier 16 is hinged to a maximum of 50°. The offset angle, and in the open position of the actuator jaws, the distance from the center of the carrier pivot pin 70 to the distal tips of the actuator jaws 18, 20 will be on the order of 50 mm.

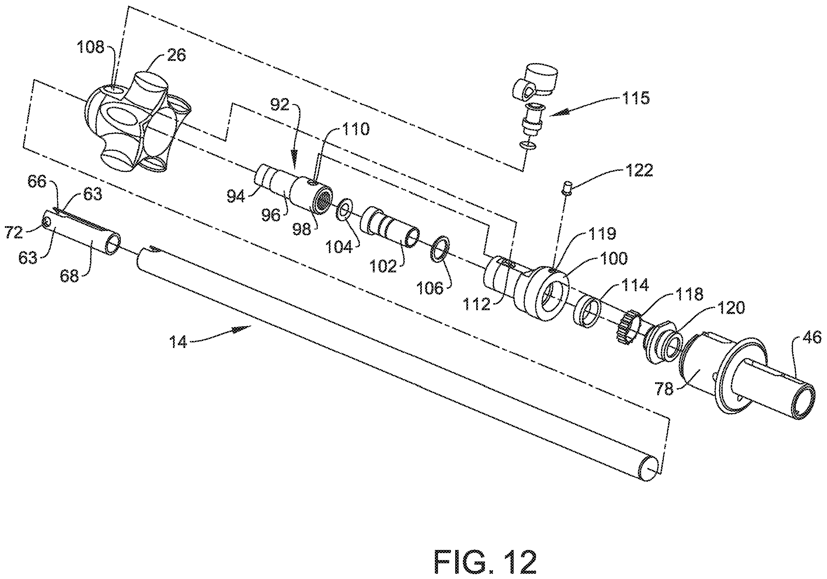

Referring now to FIG. 12, the neck 94 of the stepped collar 92 with a reduced diameter is provided and fixed in the proximal end of the barrel 14. The shoulder of the middle diameter portion 96 abuts against the proximal end of the barrel 14. The larger diameter proximal portion 98 of the collar 92 is received in the hole of the main sleeve 100. The transition sleeve 102 is also located in the hole of the main sleeve, where the washer 102 abuts the proximal end of the collar 92 and the other washer 106 is located on the sleeve 102. When assembled, flush channels are provided through aligned openings 108, 110, and 112 in barrel button 26, stepped collar 92, and main sleeve 100, respectively. The flushing socket assembly 115 is fitted in the opening 108 of the barrel button.

The washer 114, the ring gear 118, and the end collar 120 are located in the proximal end of the hole of the main sleeve 100. It should be noted that when the barrel knob 26, the stepped collar 92, and the main sleeve 100 rotate in unison, the end collar 120 and the ring gear 118 remain stationary. When the main sleeve 100 rotates, the teeth of the ring gear 118 are engaged by the ball stopper latch 122 located in the socket 119 of the main sleeve 100, thereby increasing the barrel 14 by a yieldable and audible limit stopper Rotation.

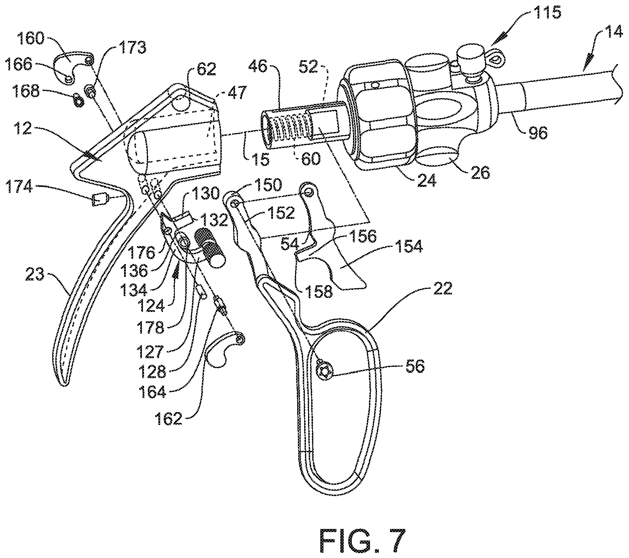

A safety mechanism is provided to ensure that accidental actuation of the actuator jaws 18, 20 is prevented. 7 (shown in FIG. 7 that the grip 22 of the handle portion 12 is in an automatic locking position), the lock/release lever 124 includes a pair of knurled fingers at the distal end of the integrated arm 127 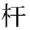 126. The upper part of the lock/release lever 124 is located in the hollow area of the seat 23. The lock/release lever 124 is configured to pivot about a pin 128 that extends through the seat 23.

Protruding distally from the upper portion of the lock/release lever 124 is an integrated latch 130 having a concave abutting end 132. A bend 134 having a distal end surface 136 and a proximal end surface 176 integrally engages the arm 127 and the latch 130. It should be noted that the plate 154 includes a proximally protruding impact leg 156 having abutting ends 158.

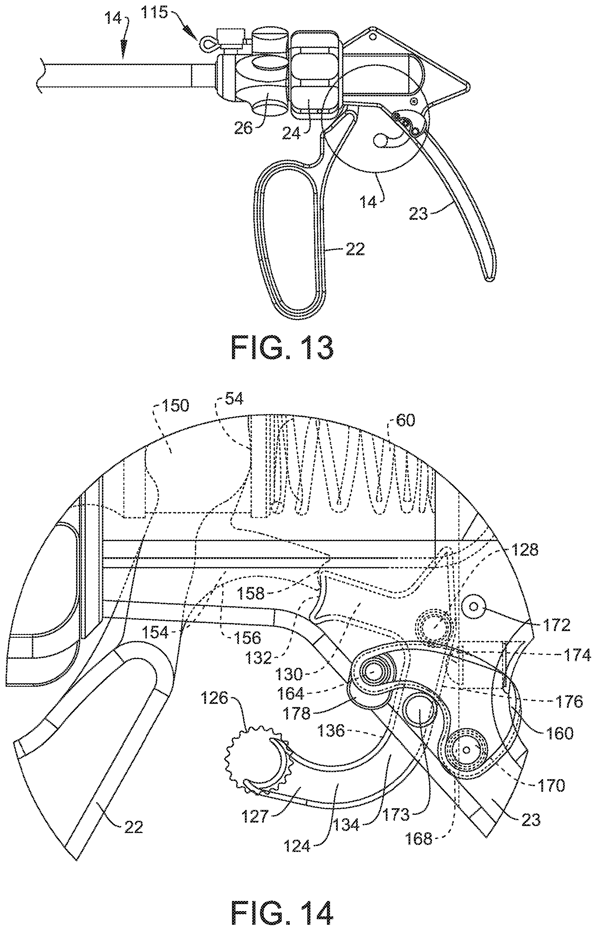

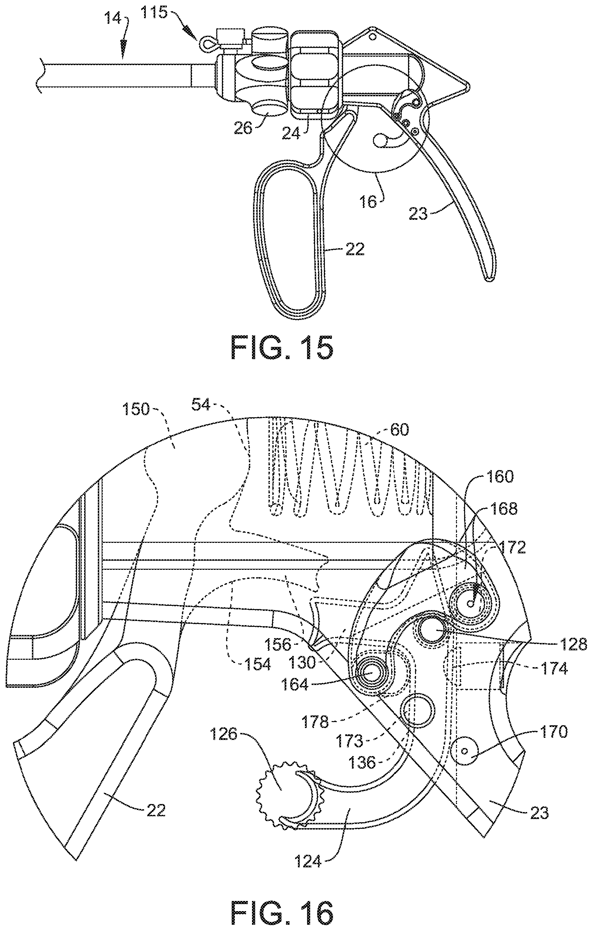

The left toggle arm 160 and the right toggle arm 162 are fixed to opposite ends of the camshaft 164 for use in the automatic locking position shown in FIG. 14 and the unlocking shown in FIG. 16 along the opposite sides of the handle portion 12 Rotate between positions. The inner face of each toggle arm includes a recess 166 that receives a spherical latch 168. The ball latch 168 is located in the stopper 170 in the locked position, and the ball latch 168 is located in the stopper 172 when the toggle arm is in the unlocked position. When the ball latch is located in the stopper 170, the stop pin 173 prevents the toggle arm from rotating further.

As shown in FIG. 14, when the toggle arms 160, 162 are in the automatic locking position, due to the abutting contact between the corresponding abutting end 132 and the abutting end 158, the grip 22 is prevented from being close Movement in the end direction. The spring-loaded spherical component 174 is located in the proximal end of the handle portion 12 and abuts the proximal surface 176 of the curved portion 134, while the cam 178 fixed on the camshaft 164 abuts the distal surface 136 of the curved portion 134.

The surgeon can unlock the grip 22 by grasping the knurled finger 126 and pulling the locking/release rod 124 toward the proximal end against the bias of the spring-loaded spherical assembly 174, thereby enabling the locking/release rod 124 rotates around pin 128, whereby the striker leg 156 will no longer be aligned with the latch 130. The grip 22 can then be pulled toward the proximal end, for example, to open the actuator jaws 18, 20 to engage or release the ligation clip. When the knurled finger 126 is released, the lock/release lever 124 returns to its position in FIG. 14 due to the bias of the spring ball assembly 174 against the end surface 176.

In Figure 16, the safety mechanism components are shown disengaged so that the grip 22 can be pulled without the need to rotate the lock/release lever 124. In order to disengage the safety mechanism, as shown in FIG. 16, the toggle arms 160, 162 rotate counterclockwise with the camshaft 164 by about 90° (as can be seen from FIGS. 13 to 15) until the ball latch 168 is in the stop Item 172. The rotation of the camshaft 164 causes the cam 178 to be supported against the distal surface 136 and causes the locking/release lever 124 to rotate counterclockwise against the bias of the spring ball assembly 174 to its position of FIG. 16, in the position of FIG. 16. In this case, the impact leg 156 is no longer aligned with the latch 130, and the grip 22 can be freely actuated repeatedly throughout the procedure.

The specific combinations of elements and features in the above-mentioned embodiments are only exemplary; the interchange and replacement of these teachings in this application with other teachings is also explicitly considered. As those skilled in the art will recognize, without departing from the scope of the claimed invention, those skilled in the art can think of variations, modifications and other embodiments of the content described herein.

Having described the preferred embodiments of the present invention, it will now be apparent to those of ordinary skill in the art that other embodiments incorporating this concept may be used. Moreover, those of ordinary skill in the art will understand that the embodiments of the present invention described herein may be modified to adapt to and/or follow the changes and improvements of applicable technologies and standards indicated herein.

Without departing from the scope of the claimed invention, those of ordinary skill in the art can think of variations, modifications and other embodiments of the content described herein. Therefore, it can be understood that these embodiments should not be limited to the disclosed embodiments, but should only be limited by the scope of the appended claims

## Claims

1. A surgical appliance (10) comprising an elongate barrel (14), a carrier (16) secured in a distal end of the barrel, effector jaws (18, 20) mounted to the carrier, a rod (32) positioned within the barrel, a distal end of the rod operatively connected to the effector jaws for opening and closing the effector jaws, a handle (12) at the proximal end of the barrel, the handle including a linear actuator (46), a proximal end of the rod being operatively connected to the linear actuator, the linear actuator having a slot, the rod is fixed to a ball (44), the linear actuator having a socket (48) with the ball seated in the socket, the handle further including a pivotally mounted grip (22), the grip including a primary leg, the primary leg being received in the slot, whereby pivotal movement of the grip results in linear movement of the rod and movement of the effector jaws relative to one another; wherein the surgical appliance further includes a barrel rotation knob (26) and a sleeve (100) the barrel rotation knob, the barrel and the sleeve being coupled to rotate in unison, a ring gear (118) received in the sleeve, the ring gear being fixed against rotation with respect to the sleeve, the handle further including a detent latch (122) extending radially through the sleeve, the detent latch engaging the ring gear, whereby rotation of the barrel rotation knob is incremented with yieldable and audible limit stops.

2. The surgical appliance in accordance with Claim 1 wherein the handle includes a carrier articulation knob (24) and a safety mechanism for automatically preventing the effector jaws from closing as a result of inadvertent operation of the grip.

3. The surgical appliance in accordance with Claim 1 or 2 wherein each effector jaw has a cam slot (36, 37) associated with a pin (34) which is operatively connected to the distal end of the rod, the pin extending transversely through the cam slots, whereby linear movement of the rod moves the effector jaws relative to one another.

4. The surgical appliance in accordance with Claims 1, 2 or 3 further including a return spring (60) which bears against the linear actuator to urge, the linear actuator and the rod in a distal direction such that the effector jaws are normally open.

5. The surgical appliance in accordance with Claim 2 or Claims 3 or 4, when dependent on Claim 2, wherein the safety mechanism includes a lever (124) rotatable from a lock position, wherein the grip is precluded from rotating, to an unlock position, wherein the grip is not precluded from rotating, the safety mechanism further including a spring element biasing the lever toward the lock position; and a cam which is rotatable to bear against a surface of the lever to maintain the lever in the unlock position against the bias of the spring element.

6. The surgical appliance in accordance with Claim 1 wherein the primary leg includes a laminate of panels (150, 152, 154) having cam surfaces (54), the cam surfaces being received in the slot.

## Patentansprüche

1. Ein chirurgisches Gerät (10), umfassend einen länglichen Lauf (14), einen Träger (16), der an einem distalen Ende des Laufs angebracht ist, Greifzangenbacken (18, 20), die an dem Träger montiert sind, eine Stange (32), die innerhalb des Laufs positioniert ist, ein distales Ende der Stange ist betriebsbereit mit den Greifzangenbacken für das Öffnen und Schließen der Greifzangenbacken verbunden, einen Handgriff (12) an dem proximalen Ende des Laufs, der Handgriff beinhaltet einen linearen Aktuator (46), ein proximales Ende der Stange ist betriebsbereit verbunden mit dem linearen Aktuator, der lineare Aktuator weist eine Nut auf, die Stange ist an einer Kugel (44) befestigt, der lineare Aktuator hat eine Aufnahme (48), wobei die Kugel in der Aufnahme sitzt, der Handgriff beinhaltet ferner einen drehbar montierten Griff (22), der Griff beinhaltet einen ersten Schenkel, der in die Nut eingesteckt ist, wodurch eine Drehbewegung des Griffs zu einer linearen Bewegung der Stange und Bewegung der Greifzangenbacken im Verhältnis zueinander führt; wobei das chirurgische Gerät ferner einen Lauf-Drehknopf (26) und eine Hülse (100) beinhaltet, der Lauf-Drehknopf, der Lauf und die Hülse sind so gekoppelt, dass sie sich im Einklang miteinander drehen, einen Zahnkranz (118), der von der Hülse aufgenommen wird, der Zahnkranz ist gegen Drehung im Verhältnis zu der Hülse fixiert, der Griff beinhaltet ferner eine Sperrklinke (122), die sich radial durch die Hülse erstreckt, die Sperrklinke greift in den Zahnkranz ein, wodurch die Drehung des Lauf-Drehknopfes mit nachgiebigen und hörbaren Begrenzungsanschlägen erhöht wird.

2. Chirurgisches Gerät nach Anspruch 1, wobei der Handgriff einen Träger-Gelenkknopf (24) und einen Sicherheitsmechanismus beinhaltet, um zu verhindern, dass sich die Greifzangenbacken automatisch infolge einer unbeabsichtigten Betätigung des Griffs schließen.

3. Chirurgisches Gerät nach Anspruch 1 oder 2, wobei jede Greifzangenbacke eine Nockennut (36, 37) in Verbindung mit einem Stift (34) aufweist, die betriebsbereit mit dem distalen Ende der Stange verbunden ist, der Stift erstreckt sich quer durch die Nockennuten, wodurch die lineare Bewegung der Stange die Greifzangenbacken im Verhältnis zueinander bewegt.

4. Chirurgisches Gerät nach Anspruch 1, 2 oder 3, das ferner eine Rückstellfeder (60) beinhaltet, die gegen den linearen Aktuator wirkt, um den linearen Aktuator und die Stange in eine distale Richtung zu drängen, so dass die Greifzangenbacken normalerweise geöffnet sind.

5. Chirurgisches Gerät nach Anspruch 2 oder Anspruch 3 oder 4, abhängig von Anspruch 2, wobei der Sicherheitsmechanismus einen Hebel (124) beinhaltet, der aus einer Verriegelungsposition, in der der Griff von der Drehung ausgeschlossen ist, in eine Entriegelungsposition drehbar ist, in der der Griff nicht von der Drehung ausgeschlossen ist, wobei der Sicherheitsmechanismus ferner ein Federelement beinhaltet, das den Hebel in Richtung der Verriegelungsposition vorspannt, und einen Nocken umfasst, der drehbar ist, um gegen eine Oberfläche des Hebels zu drücken, um den Hebel gegen die Vorspannung des Federelements in der Entriegelungsposition zu halten.

6. Chirurgisches Gerät nach Anspruch 1, wobei der erste Schenkel Laminatplatten (150, 152, 154) mit Nockenflächen (54) beinhaltet, wobei die Nockenflächen von der Nut aufgenommen werden.

## Revendications

1. Appareil chirurgical (10) comprenant un cylindre allongé (14), un support (16) fixé à une extrémité distale du cylindre, des mâchoires effectrices (18, 20) montées sur le support, une tige (32) positionnée à l'intérieur du cylindre, une extrémité distale de la tige reliée fonctionnellement aux mâchoires effectrices destinée à ouvrir et à fermer les mâchoires effectrices, une poignée (12) à l'extrémité proximale du cylindre, la poignée comportant un actionneur linéaire (46), une extrémité proximale de la tige étant reliée fonctionnellement à l'actionneur linéaire, l'actionneur linéaire comporte une fente, la tige est fixée à une bille (44), l'actionneur linéaire présentant une douille (48), la bille étant logée dans la douille, la poignée comportant en outre une partie de préhension montée pivotante (22), la partie de préhension comportant une branche principale, la branche principale étant reçue dans la fente, grâce à quoi le mouvement pivotant de la partie de préhension entraîne un mouvement linéaire de la tige et un mouvement des mâchoires effectrices l'une par rapport à l'autre ; dans lequel l'appareil chirurgical comporte en outre un bouton de rotation du cylindre (26) et un manchon (100), le bouton de rotation du cylindre, le cylindre et le manchon étant accouplés pour tourner à l'unisson, une roue dentée (118) reçue dans le manchon, la roue dentée étant immobilisée en rotation par rapport au manchon, la poignée comportant en outre un loquet de cran d'arrêt (122) s'étendant radialement à travers le manchon, le loquet de cran d'arrêt s'engage dans la roue dentée, grâce à quoi la rotation du bouton de rotation du cylindre est incrémentée et comporte des butées de fin de course flexibles et audibles.

2. Appareil chirurgical selon la revendication 1, dans lequel la poignée comporte un bouton d'articulation du support (24) et un mécanisme de sécurité destiné à empêcher automatiquement les mâchoires effectrices de se fermer à la suite d'un fonctionnement accidentel de la partie de préhension.

3. Appareil chirurgical selon la revendication 1 ou 2, dans lequel chaque mâchoire effectrice présente une fente de came (36, 37) associée à une broche (34) qui est reliée fonctionnellement à l'extrémité distale de la tige, la broche s'étendant transversalement à travers les fentes de la came, grâce à quoi le mouvement linéaire de la tige déplace les mâchoires effectrices l'une par rapport à l'autre.

4. Appareil chirurgical selon les revendications 1, 2 ou 3, comportant en outre un ressort de rappel (60) qui s'appuie contre l'actionneur linéaire pour pousser l'actionneur linéaire et la tige dans une direction distale de sorte que les mâchoires effectrices sont normalement ouvertes.

5. Appareil chirurgical selon la revendication 2 ou les revendications 3 ou 4, lorsqu'elles dépendent de la revendication 2, dans lequel le mécanisme de sécurité comporte un levier (124) pouvant tourner depuis une position de verrouillage, dans lequel la partie de préhension est empêchée de tourner, vers une position de déverrouillage, dans lequel la partie de préhension n'est pas empêchée de tourner, le mécanisme de sécurité comportant en outre un élément de ressort sollicitant le levier vers la position de verrouillage ; et une came qui peut tourner pour porter contre une surface du levier afin de maintenir le levier dans la position de déverrouillage contre la sollicitation de l'élément de ressort.

6. Appareil chirurgical selon la revendication 1, dans lequel la branche principale comprend un stratifié de panneaux (150, 152, 154) présentant des surfaces de came (54), les surfaces de came étant reçues dans la fente.
